# EUROPEAN PATENT SPECIFICATION

(11) **EP 3 393 411 B1**
(45) Date of publication and mention of the grant of the patent: **16.09.2026**
(21) Application number: 16876986.7
(22) Date of filing: 25.11.2016
(51) Int. Cl.: A61F 6/04, B29L 31/00

(54) **COATED CONDOM**
BESCHICHTETES KONDOM
PRÉSERVATIF REVÊTU

(30) Priority: 21.12.2015 US 201514976543
(43) Date of publication of application: 31.10.2018
(73) Proprietor: LifeStyles Healthcare Pte. Ltd., Singapore 068877 (SG)
(72) Inventor: NGUYEN, KC, Dothan, AL 36303 (US); PATTANAPRADIT, Utain, Amphur Muang, Surat Thani (TH); NGOWPRASERT, Chayapon, A. Phunphin Surat Thani 84130 (TH); PONGTHANOMSAK, Chayaporn, A. Phunphin Surat Thani 84130 (TH)
(74) Representative: Maiwald GmbH
(86) International application number: PCT/AU2016/000386
(87) International publication number: WO 2017/106897

(56) References cited:
- WO-A1-2015/188216
- US-A- 5 878 747
- US-A1- 2002 103 414
- US-A1- 2004 118 408
- US-A1- 2005 076 917
- US-A1- 2005 076 917
- US-A1- 2006 169 287
- US-A1- 2012 294 820
- US-A1- 2014 326 250
- US-A1- 2015 320 586

## Description

### Field

Embodiments of the present invention generally relate to condoms and, more particularly, to a packaged coated condom comprising at least one silicone-based lubricant and methods of fabricating coated condoms.

### Background

A condom comprises an elastomeric layer in a generally tubular shape, with an open end and a closed end, to provide physical barriers against the transmission of bodily fluids, viruses, and the like. A typical condom is approximately 180 millimeters in length and has a wall thickness of approximately 0.06 mm. Also, the typical condom has a tip, such as reservoir for semen, that is approximately 25 millimeters in length at a closed end of a condom and a bead or "ring," which is approximately 1.5 millimeters in thickness, at an open end of the condom. With the foregoing dimensions in view, a rolled condom must be rolled approximately fifteen times around its bead for a rolled length of approximately 2,54 cm (1 inch) at packaging.

Some condoms comprise a coating, such as a lubricant, located thereon. Typically, after the condom is rolled, the coating is introduced near the tip of the rolled condom. Such processes are, for example, described in WO 2015/188216 A1, US 2005/076917 A1, and US 2002/103414 A1. However, because the coating, even a lubricant, must migrate through approximately fifteen rolls of elastomeric material and the material is stretched while being tightly rolled, lubricant does not flow along all the rolls and lubricant coverage along the full length of the condom cannot occur. Specifically, the lubricant does not migrate to other areas of condoms, e.g., from a tip to a bead. Typically, even after several months or years, only 50% of the length of condoms is lubricated (i.e., 50% lubricant migration achieved). Additionally, much of the lubricant remains on the foil packaging instead of instead of being dispersed throughout an exterior surface of the condom.
From the consumer perspective, this phenomenon could be translated as low lubricant/condom dryness issue due to not enough lubricant on the condom.

Accordingly, there exists a need for a condom that is fully coated along its length, and a method of manufacturing a fully coated condom, that allows the coating to migrate throughout the entire condom much easier and faster and thus prevent, for example, the low coating and/or lubricant/condom dryness phenomenon.

### Object of Invention

It is an object of the present invention to substantially overcome or at least ameliorate one or more of the disadvantages of the prior art, or to at least provide a useful alternative.

### Summary of Invention

The invention is defined by the appended claims. Embodiments according to the invention include packaged coated condoms in which the lubricant coating substantially covers an entire length of the condom when packaged, and methods for manufacturing such condoms, substantially as shown in and/or described in connection with at least one of the figures, as set forth more completely in the claims. Various advantages, aspects, and novel features of the present disclosure will be more fully understood from the following description and drawings.

### Brief Description of the Drawings

So that the manner in which the above recited features of the present invention can be understood in detail, a more particular description of the invention, briefly summarized above, may be had by reference to embodiments, some of which are illustrated in the appended drawings. It is further understood that, where possible, identical reference numerals have been used to indicate comparable elements that are common to the figures.
Figure 1 depicts a perspective view of a rolled condom according to embodiments of the invention;
Figure 2 depicts a perspective view of an unrolled condom, according to embodiments of the invention;
Figure 3 depicts a perspective view of an unrolled, coated condom, according to embodiments of the invention; and
Figure 4 depicts a flow diagram of a method for manufacturing a coated condom, according to embodiments of the invention.

### Detailed Description of the Preferred Embodiments

Embodiments of the present invention generally relate to fully coated condoms, and methods for manufacturing fully coated condoms as defined in the appended claims. In embodiments consistent with the present invention, a fluidic coating material comprising one or more silicone-based lubricants is advantageously disposed on substantially all external surfaces of the condom. That is, the condom is fully coated along the entire length of the condom. In embodiments consistent with the present invention, methods for manufacturing fully coated condoms are described herein which include the application of a fluidic coating material to the condom prior to rolling the condom. By applying the fluidic coating material prior to rolling the condom, the fluidic coating material is able to migrate throughout the entire condom much easier and faster and thus prevent a low coating condom dryness phenomenon.

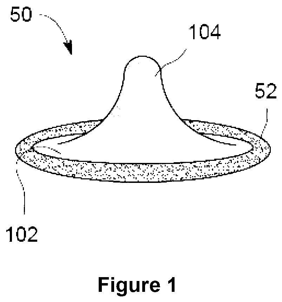

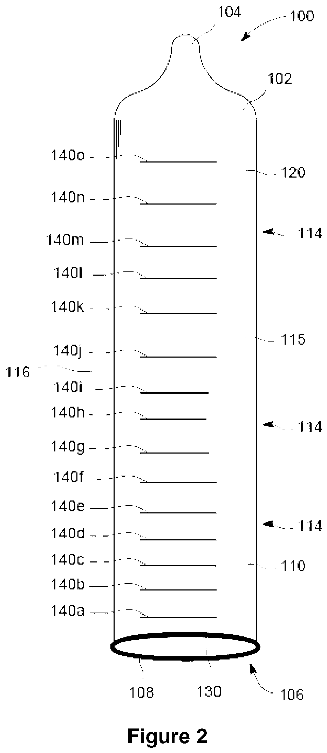

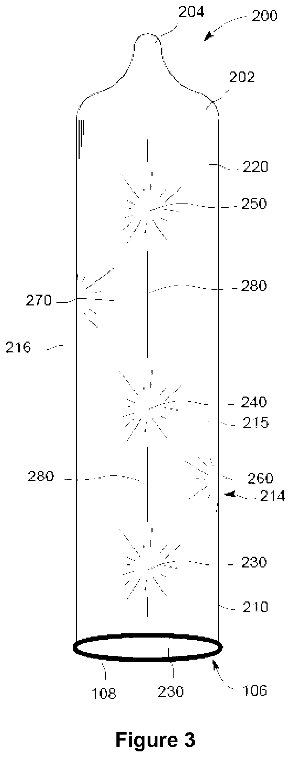

Figure 1 depicts a perspective view of a rolled condom. The rolled condom 50 comprises a rolled portion 52 and a tip 102 further comprising a reservoir 104. The rolled condom 50 comprises an elastomeric material, such as a natural latex rubber, a synthetic polyisoprene material, a polyurethane material, polymeric blends, or a like polymeric material used in condoms. The rolled condom 50 configuration shown in Figure 1 is typically how condoms are configured for packaging. The rolled condom 50 also comprises a tubular shaft and a bead as will be described further below with respect to Figures 2 and 3. Depending on the length of a condom, a rolled portion 52 may include from about 15 to about 17 layers/rolls. For example, a condom having a straight shaft and a 53mm width and approximately 185mm in length yields sixteen layers.

Figure 2 depicts a perspective view of an unrolled condom 100 having a front end 120, having a tip 102, and a reservoir 104, a rear end 110 having an opening 106 for receiving a penis, and a tubular shaft 116 disposed between the front end 120 and the rear end 110, and optionally comprises a bead 108, according to embodiments of the invention. The tubular shaft 116 comprises a middle portion 115 and an exterior shaft surface 114. The condom 100 further comprises a reservoir 104 disposed adjacent to the tip 102 and an internal surface 130. Figure 2 further depicts roll lines 140a-140o. The roll lines 140a-140o are not to be contemplated as physical structure but rather are indicative of approximate demarcations of complete revolutions as the bead 108 is rolled and the condom is rolled into a rolled configuration. As shown, the distance between adjacent roll lines 140 increases as the condom 100 is rolled from the bead 108 to roll line 140a ... 140o, as the thickness of a rolled portion increases with each successive roll.

Figure 3 depicts a perspective view of an unrolled, lubricated condom 200, according to embodiments of the invention. The unrolled condom 200 comprises a front end 220, and a tip 202 and a reservoir 204 adjacent to the front end 220, a rear end 210 further comprising a bead 108 and having an opening 106 for receiving a penis. A tubular shaft 216 is disposed between the front end 220 and the rear end 210. The tubular shaft 216 further comprises a middle portion 215, and an exterior shaft surface 214. In some embodiments, the condom length is approximately 180-220 mm. In some embodiments, the cross-sectional thickness of the condom 200 is about 0.04-0.12 mm thick and, for at least one exemplary embodiment of the invention, 0.06 mm thick.

The condom 200 further comprises one or more coating spots 230, 240, 250, 260, and/or 270 that comprise one or more fluidic coating materials comprising one or more silicone-based lubricants, disposed on the exterior shaft surface 214. Although multiple coating spots are shown in Figure 3, in some embodiments, a single coating spot or area may be used for application of the fluidic coating prior to rolling. As shown, the one or more coating spots are disposed on the exterior shaft surface 214 near the front end 220, near the rear end 210, and near the middle portion 215 as well as at areas disposed therebetween. The coating spots 250, 240, and 230 are shown as being disposed generally in line with a longitudinal axis 280 that runs from the front end 220 and the rear end 210. The coating spots 260 and 270 are show as disposed offset from the longitudinal axis 280. In addition, the condom 200 may further comprise coating spots on a side opposite the longitudinal axis 280, i.e., 180 degrees around a circumference of the condom 200. It is to be further understood that the condom 200, before rolling, may comprise one coating spot or a plurality of coating spots. Further still, the condom 200 may comprise a fluidic coating material comprising one or more silicone-based lubricants that covers substantially all of the exterior shaft surface 214 before rolling. Irrespective of the number of coating spots applied before rolling, embodiments according to the invention comprise condoms having a fluidic coating material comprising one or more silicone-based lubricants along the entire length of the condom after rolling, so that the exterior shaft surface 214 of the condom 200 is substantially covered to form a coated condom. Embodiments of the invention comprise condoms having a fluidic coating material in amounts ranging from 50-1000mg per coated condom. At least one exemplary embodiment according to the invention comprises between 150-300mg of lubricant per coated condom.

Embodiments according to the invention comprise further coatings of several kinds. For example, water based lubricants, gels, and the like, as well as flavorings, scents, and/or active ingredients, e.g., sensitizing agents that provide warming sensations, cooling sensations, and/or tingling sensations, desensitizing agents, and/or spermicides. At least some embodiments according to the invention include a coated condom having at least one active ingredient, and/or additional lubricant, displaced in or near the tip after rolling, in addition to the lubricant disposed on the condom prior to rolling. Active ingredients comprise medicines, such as anti-virals, anti-microbials, and/or anti-fungals, and vasodilators such as niacin, sildenafil citrate, and/or nitroglycerin and/or male desensitizing agents such as benzocaine, and warming liquids such as caffeine or menthol, and/or the like, and/or spermicides such as nonoxynol-9. Because some active ingredients may be disposed within the tip, less of the active ingredient(s) is needed because the active ingredient(s) remains localized and a higher concentration is therefore delivered to the tip of the penis, where it is needed, during or before sexual contact. For example, typically, benzocaine is administered in a 3.0 to 7.5% in a water-soluble base medium. Therefore, a typical dosage within a condom is 300-400 mg at 4.5% concentration. Because the majority of the active ingredient is localized at the tip, less dosage is needed, e.g., 200-300 mg to provide efficacy without overdosing.

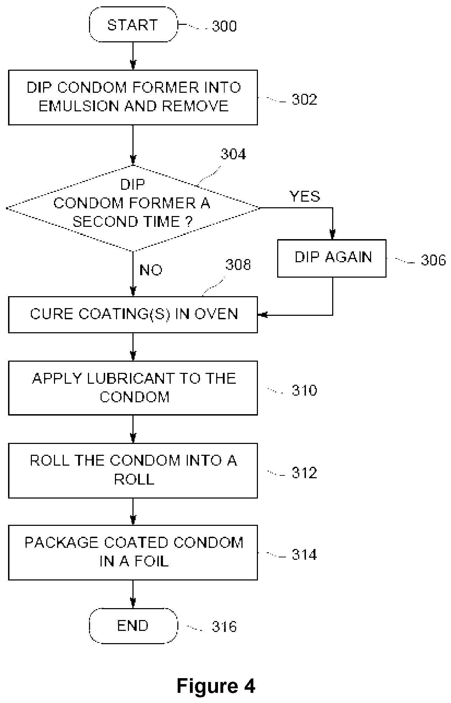

Figure 4 depicts a flow diagram of a method for manufacturing a fully coated condom, according to embodiments of the invention. The method starts at step 300 and proceeds to step 302, at which point a condom former is dipped into an emulsion, such as a tank of polymeric, elastomeric, or latex emulsion, forming a latex coating on the former. The words polymeric, elastomeric, and latex may be used interchangeably herein. At step 304, a decision is made whether to dip the former, now having a latex coating thereon, into the tank of polymeric, elastomeric, or latex emulsion a second time. If the answer is yes, a second latex coating is disposed thereon at step 306 and proceeds to step 308, at which point the latex coating(s) on the condom former, comprised of the latex emulsion or composition, is cured at a temperature of approximately, for example, 90-140°C for approximately 5-15 minutes in an oven to form a condom. At step 310, the fluidic coating material is applied to the condom. In some embodiments, the fluidic coating may be disposed by spraying, dipping, sponging, rolling in lubricant, or otherwise dosing. As used herein, sponging means contacting the condom with a sponge or other absorbent/adsorbent material containing the fluidic coating material, etc., wherein the fluidic coating material is transferred to the condom. It is to be further noted that the spraying, sponging, dipping, rolling in fluidic coating material, or otherwise dosing steps need not cover the entire exterior surface of the condom immediately. In some embodiments, the fluidic coating material may be applied to a single coating spot, such as the rear end 210 of the condom proximate the bead 108 (e.g., coating spot 230). It is to be understood that applying fluidic coating materials at one or more coating spots near the bead 108 promotes the migration of the fluidic coating material(s) to cover substantially all of the condom. In other embodiments, the fluidic coating material may be applied to one or a plurality of coating spots along the length of the condom in various areas before rolling.

At 312, the condom is rolled to form a fully coated condom having a fluidic coating, comprising at least one silicone-based lubricant, disposed along essentially the length of the condom, so that all of the exterior surface of the condom comprises lubricant. Since the lubricant is applied to condom before rolling, the lubricant migrates to all areas of the condom if not immediately, within hours. Specifically, the action of rolling the condom promotes the migration of the lubricant to substantially all of the length of the condom. That is, rolling the condom pushes/forces the lubricant to move forward from the rear end 210 proximate the opening 106 towards the front end 220 proximate the tip 202. At step 314 the lubricated, rolled condom may be packaged in a foil packaging. At step 316, the method ends.

It is to be understood that, in some embodiments according to the invention, each step of the method is performed. Also, in some embodiments, some steps are omitted and/or additional steps are performed. For example, any method herein can include a step for adding, for example, additional lubricant, flavoring, scent, active ingredient, etc., after the rolling step or other optional steps for cleaning a condom former, for example, with brushes, such as nylon brushes, and/or pre-heating the former, for example, with hot air or within an oven at a temperature of approximately 35-60°C. Also, in at least one or more embodiments, a strong or weak coagulant component or solution, as is known to those in the art, may be disposed onto the former. If a coagulant solution is disposed on the former, it is optionally dried, for example, at 50-70°C for approximately 2-5 minutes. The condom may then be as be washed, leached, etc., as is known to those in the art before the lubricant is applied thereto. The method may include a step for stripping the condom from the condom former. In at least one embodiment according to the invention, the inverting and rolling steps are performed by, for example, an air jet or compressed air, such as at 200-400 kPa (2-4 bar) of pressure, sprays the condom and strips the condom at the same time. Optionally, the condom may be stripped partially from the former and a roller used to strip the condom from the condom former. Compressed air may also promote the migration of the lubricant to further areas of the condom.

Coated condoms described herein are made using polymeric, elastomeric, and/or latex emulsions or compositions, such as natural rubber latex, synthetic polyisoprene, polyurethane, and other elastomeric materials for emulsions or compositions, and/or blends thereof, as are described herein comprises vulcanizing agents, activators, accelerators, antioxidants, stabilizers, thixotropic agents and/or the like as are known to those in the art. At least one exemplary embodiment of a formula for an emulsion or composition according to embodiments of the invention comprises potassium hydroxide, ammonium solutions, and/or the like for diluting the emulsion or composition, adjusting the pH, and the like and/or surfactants, such as a polysorbate, for example, TWEEN^{®} 20, to stabilize the emulsion or composition.

Additionally, the temperature of the emulsion or composition may be controlled, for example, the temperature may be from approximately 20°C to approximately 30°C during the dipping process. Also, embodiments of the polymeric compositions according to the invention comprise additives to control or modify the properties of the composition, such as the viscosity of the composition as well as the physical properties, for example, lubricity, tensile strength, puncture resistance, and the like, of condoms formed therefrom. The viscosity of the compositions according to embodiments of the invention is, for example, approximately 20 to approximately 60 mPa*s (centipoises).

As used herein, the terms polymeric, elastomeric, thermoplastic elastomer, latex, and rubber are used interchangeably to describe material, such as a polymeric composition, used to form coated condoms in accordance with embodiments of the invention. Emulsions or compositions include elastomeric compositions, polymeric compositions, latex compositions, and natural rubber compositions, synthetic compositions, and/or blends or mixtures thereof. The term "natural rubber latex" as used in this disclosure encompasses cured elastomeric material sourced from Hevea brasiliensis (the traditional rubber tree), non-Hevea rubber such as Parthenium argentatum (guayule), sunflower, goldenrod, and the like, as well as genetically modified variations of these or other biological sources. In some embodiments of the invention, condoms comprise the pre-vulcanized and post-vulcanized latex composition as disclosed in commonly-assigned US Patent No. 8,087,412, which serves as background to the present invention.
In some embodiments of the invention, synthetic polyisoprenes, polychloroprenes, carboxylated butadiene-nitriles, polyurethanes or polyurethane-polyurea copolymers, or combinations thereof are used.

The total solids content range of the emulsions or compositions, which may include a natural color or another color, range from about 28% to about 70%. At least one exemplary embodiment according to the invention comprises a composition having a total solids content of approximately 53%. Moreover, processing aids, additives, rheological additives, stabilizers, and the like, known to those in the art, may be incorporated into any emulsion or composition.

Furthermore, embodiments according to the invention comprise compositions having colorants and/or pigments, and further include glow-in-the-dark or fluorescent colorants or pigments. For example, at least one pigment according to embodiments of the present invention is a Quinacridone, such as Colanyl^{®} Red E3B 130 manufactured by the Clariant Corp., or a Phthalocyanine, such as Colanyl^{®} Blue A2R 131 also manufactured by the Clariant Corp., or combinations thereof. Examples of glow-in-the-dark pigments include photoluminescent pigments, such as SP-6-B distributed by Farben Technology but are not limited thereto.

The coated condoms described herein may be manufactured using a condom former, which may be a smooth former or, alternatively, a former having depressions on the surface, e.g., a textured former, which create ribs, studs, and the like, on an interior surface of a condom. In one or more embodiments, the condom former may include a tubular body having a first end and a second end. The tubular body may have an overall shape that is similar to the shape of a penis, thereby resulting in the polymeric layer of the condom described above. The tubular body of the condom former may include a base segment that is disposed adjacent to the first end and extends from the first end toward the second end. In one or more embodiments, the second end is utilized to form a closed end of the condom described above, while the first end of the former is utilized to form an open end and a base portion of the condom described above. The condom former according to one or more embodiments may be formed from glass, borosilicates, ceramic materials, metallic materials, and/or other materials known in the art. In one or more embodiments, at least one method includes providing a former comprising an axial length, a circumference, and a plurality of depressions, ribs, or protrusions disposed along at least a portion of the length and around or along the circumference of the former as is disclosed in commonly assigned US Pub. No. 2012/0073580.

Also, in some embodiments, one or more coagulant or primer solutions are disposed onto the condom former before dipping into an emulsion or composition, and in some embodiments, coagulant or primer solutions are not disposed onto the condom former. Coagulant or primer solutions comprise concentration ranging from about 1% to about 50% by weight, and may include a natural color or another color. In some embodiments of the present invention, the coagulant concentration is about 5% by weight. According to some embodiments, the coagulant solution may contain Group I metal salts, Group II metal salts, or combinations thereof, and wetting agents ranging from 0.1-0.2% by weight in an aqueous or alcoholic solution. In some embodiments of the invention, the coagulant is an aqueous solution comprising 3.5% Calcium Nitrate and 96.5% water, e.g., a strong coagulant.

In at least one exemplary embodiment according to the invention, the coagulant solution comprises a 3-15% Calcium Nitrate or other Calcium salt, 2-10% Calcium Carbonate, and a small amount of surfactant and anti-foam agent, as are known to those in the art. Furthermore, at least one exemplary embodiment replaces Calcium Carbonate with a powder-free coagulant, such as a stearate, such as Calcium Stearate, so that post-washing steps may be omitted. Other suitable strong coagulants known to those in the art may also be used, such as calcium chloride. Weak coagulants include acetic acid, formic acid, and/or other weak acids.

Embodiments according to the invention comprise packaging made of foils. At least one exemplary embodiment according to the invention comprises environmentally friend composite foils, such as the technologies disclosed in US Pub. No. 2015/0217537.

## Claims

1. A packaged coated condom, comprising:
a condom (100) in a rolled configuration comprising:
a tubular shaft (116) having a tip (102) on a closed end of the tubular shaft (116) and an open end (106) opposite the closed end on the tubular shaft (116); and
a coating material disposed along a length of the tubular shaft (116) that extends from the open end (106) of the tubular shaft (116) to the tip (102) on the closed end of the tubular shaft (116), wherein the tubular shaft (116) comprises a polymeric composition; and
a foil package inside which the condom (110) is packaged, **characterized in that** the coating material is fluidic and covers substantially all of an exterior surface (114) of the tubular shaft (116) and that the fluidic coating
material comprises one or more silicone-based lubricant(s), wherein the lubricant has been applied to the condom (110) prior to rolling.

2. The coated condom of claim 1, wherein an active ingredient is disposed in or near the tip, and is at least one of: an antiviral, an anti-microbial, an anti-fungal, a vasodilator, a benzocaine, caffeine, menthol, a warming liquid or gel, sensitizing agents, desensitizing agents, or spermicides.

3. The coated condom of claim 1, wherein upon sequential application of the fluidic coating, rolling, and packaging of the condom, the fluidic coating material fully covers substantially all of the exterior surface of the tubular shaft immediately or within hours.

4. The coated condom of claim 1, wherein 50 to 1000 mg of the fluidic coating material is disposed on the tubular shaft.

5. The coated condom of claim 1 , wherein the polymeric composition comprises at least one of natural rubber latex, synthetic polyisoprenes, polychloroprenes, carboxylated butadiene-nitriles, polyurethanes or polyurethane-polyurea copolymers, or blends thereof.

6. A method for forming the packaged coated condom (100) of any one of claims 1 to 5, comprising:
dipping a condom former into a polymeric emulsion to form a layer of the polymeric emulsion onto the condom former;
curing the layer of polymeric emulsion to form an unrolled condom (100);
applying a fluidic coating material to the unrolled condom (100) at one or more coating spots along a tubular shaft (116) of the condom (100) such that an exterior surface (114) of the unrolled condom (100) is not entirely covered, wherein the fluidic coating material comprises one or more silicone-based lubricant(s);
rolling the unrolled condom (100) into a rolled configuration, wherein the fluidic coating material migrates
such that the fluidic coating material fully covers substantially all of an exterior surface of the coated condom (100); and
packaging the fully coated condom (100) in a foil package.

7. The method of claim 6, wherein the coated condom includes a tubular shaft having a tip on a closed end of the tubular shaft and an open end opposite the closed end on the tubular shaft.

8. The method of claim 7, wherein rolling the condom pushes the fluidic coating material, forcing the fluidic coating material to migrate from the open end of the tubular shaft towards the tip on the closed end of the tubular shaft.

9. The method of claim 6, wherein the fluidic coating material is applied to a single coating spot along a tubular shaft of the unrolled condom.

10. The method of claim 6, wherein the fluidic coating material is applied to the unrolled condom by one of spraying, dipping, sponging, rolling in the fluidic coating material, or dosing.

11. The method of claim 6, wherein the emulsion comprises at least one of natural rubber latex, synthetic polyisoprenes, polychloroprenes, carboxylated butadiene-nitriles, polyurethanes or polyurethane-polyurea copolymers, or blends thereof.

12. The method of claim 6, further comprising disposing 50 to 1000 mg of the fluidic coating material onto the condom.

13. The method of claim 6, wherein upon sequential application of the fluidic coating, rolling, and packaging of the condom, the fluidic coating material fully covers substantially all of the exterior surface of the tubular shaft immediately or within hours.

## Patentansprüche

1. Ein verpacktes, beschichtetes Kondom, umfassend:
ein Kondom (100) in einer gerollten Konfiguration, umfassend:
einen tubulären Schaft (116), der auf einem geschlossenen Ende des tubulären Schafts (116) eine Spitze (102) aufweist, und ein offenes Ende (106) gegenüber dem geschlossenen Ende auf dem tubulären Schaft (116); und
ein Beschichtungsmaterial angeordnet entlang einer Länge des tubulären Schafts (116), die sich vom offenen Ende (106) des tubulären Schafts (116) bis zur Spitze (102) auf dem geschlossenen Ende des tubulären Schafts (116) erstreckt,
wobei der tubuläre Schaft (116) eine polymere Zusammensetzung umfasst; und
eine Folienverpackung in der das Kondom (110) verpackt ist, **dadurch gekennzeichnet, dass** das Beschichtungsmaterial fluidisch ist und im Wesentlichen die gesamte äußere Oberfläche (114) des tubulären Schafts (116) bedeckt
und dass das fluidische Beschichtungsmaterial ein oder mehrere Silikon-basierte(s) Gleitmittel umfasst, wobei das Gleitmittel vor dem Rollen auf dem Kondom (110) aufgebracht wurde.

2. Das beschichtete Kondom gemäß Anspruch 1, wobei ein Wirkstoff in oder in der Nähe der Spitze aufgebracht wird und mindestens eines von: einem antiviralen Mittel, einem antimikrobiellen Mittel, einem Antimykotikum, einem Gefäßerweiterer, einem Benzocain, Koffein, Menthol, einer warmen Flüssigkeit oder Gel, Sensibilisierungsmitteln, Desensibilisierungsmitteln oder Spermiziden ist.

3. Das beschichtete Kondom gemäß Anspruch 1, wobei während der aufeinanderfolgenden Anwendung von fluidischer Beschichtung, Rollen, und Verpacken des Kondoms, das fluidische Beschichtungsmaterial im Wesentlichen die gesamte äußere Oberfläche des tubulären Schafts sofort oder innerhalb von Stunden vollständig bedeckt.

4. Das beschichtete Kondom gemäß Anspruch 1, wobei 50 bis 1000 mg des fluidischen Beschichtungsmaterials auf dem tubulären Schaft aufgebracht werden.

5. Das beschichtete Kondom gemäß Anspruch 1, wobei die polymere Zusammensetzung mindestens eines von Naturkautschuklatex, synthetischen Polyisoprenen, Polychloroprenen, carboxylierten Butadien-Nitrilen, Polyurethanen oder Polyurethan-Polyharnstoff-Copolymeren oder Mischungen davon umfasst.

6. Ein Verfahren zur Herstellung des verpackten, beschichteten Kondoms (100) gemäß einem der Ansprüche 1 bis 5, umfassend:
Eintauchen eines Kondomformers in eine polymere Emulsion, um eine Schicht der polymeren Emulsion auf dem Kondomformer zu bilden;
Aushärten der Schicht der polymeren Emulsion, um ein ungerolltes Kondom (100) zu bilden;
Anwenden eines fluidischen Beschichtungsmaterials auf dem ungerollten Kondom (100) an einer oder mehreren Beschichtungsstellen entlang eines tubulären Schafts (116) des Kondoms (100), derart dass eine äußere Oberfläche (114) des ungerollten Kondoms (100) nicht vollständig bedeckt ist,
wobei das fluidische Beschichtungsmaterial ein oder mehrere Silikon-basierte(s) Gleitmittel umfasst;
Rollen des ungerollten Kondoms (100) in eine gerollte Konfiguration, wobei das fluidische Beschichtungsmaterial derart migriert, dass das fluidische Beschichtungsmaterial im Wesentlichen eine gesamte äußere Oberfläche des beschichteten Kondoms (100) vollständig bedeckt; und
Verpacken des vollständig beschichteten Kondoms (100) in eine Folienverpackung.

7. Das Verfahren gemäß Anspruch 6, wobei das beschichtete Kondom einen tubulären Schaft umfasst, der eine Spitze auf einem geschlossenen Ende des tubulären Schafts und ein offenes Ende gegenüber dem geschlossenen Ende auf dem tubulären Schaft aufweist.

8. Das Verfahren gemäß Anspruch 7, wobei das Rollen des Kondoms das fluidische Beschichtungsmaterial verdrängt, wodurch das fluidische Beschichtungsmaterial gezwungen wird vom offenen Ende des tubulären Schafts zur Spitze auf dem geschlossenen Ende des tubulären Schafts zu migrieren.

9. Das Verfahren gemäß Anspruch 6, wobei das fluidische Beschichtungsmaterial an einer einzelnen Beschichtungsstelle entlang eines tubulären Schafts des ungerollten Kondoms angewendet wird.

10. Das Verfahren gemäß Anspruch 6, wobei das fluidische Beschichtungsmaterial auf dem ungerollten Kondom durch eines von Sprühen, Eintauchen, Aufbringen mit einem Schwamm, Rollen in dem fluidischen Beschichtungsmaterial oder Dosieren aufgebracht wird.

11. Das Verfahren gemäß Anspruch 6, wobei die Emulsion mindestens eines von Naturkautschuklatex, synthetischen Polyisoprenen, Polychloroprenen, carboxylierten Butadien-Nitrilen, Polyurethanen oder Polyurethan-Polyharnstoff-Copolymeren oder Mischungen davon umfasst.

12. Das Verfahren gemäß Anspruch 6, ferner umfassend das Aufbringen von 50 bis 1000 mg des fluidischen Beschichtungsmaterials auf dem Kondom.

13. Das Verfahren gemäß Anspruch 6, wobei über das aufeinanderfolgende Anwenden der fluidischen Beschichtung, des Rollens, und des Verpackens des Kondoms, das fluidische Beschichtungsmaterial im Wesentlichen die gesamte äußere Oberfläche des tubulären Schafts sofort oder innerhalb von Stunden vollständig bedeckt.

## Revendications

1. Préservatif revêtu emballé, comprenant :
un préservatif (100) dans une configuration enroulée comprenant :
une gaine tubulaire (116) ayant une pointe (102) à une extrémité fermée de la gaine tubulaire (116) et une extrémité ouverte (106) opposée à l'extrémité fermée sur la gaine tubulaire (116) ; et
un matériau de revêtement disposé le long d'une longueur de la gaine tubulaire (116) qui s'étend de l'extrémité ouverte (106) de la gaine tubulaire (116) à la pointe (102) à l'extrémité fermée de la gaine tubulaire (116), sachant que la gaine tubulaire (116) comprend une composition polymérique ; et
un emballage en feuille à l'intérieur duquel le préservatif (110) est emballé,
**caractérisé en ce que** le matériau de revêtement est fluide et recouvre sensiblement la totalité d'une surface extérieure (114) de la gaine tubulaire (116) et **en ce que** le matériau de revêtement fluide comprend un ou plusieurs lubrifiants à base de silicone, sachant que le lubrifiant a été appliqué sur le préservatif (110) avant l'enroulement.

2. Le préservatif revêtu de la revendication 1, sachant qu'un ingrédient actif est disposé dans ou près de la pointe, et est au moins l'un de : un antiviral, un antimicrobien, un antifongique, un vasodilatateur, une benzocaïne, de la caféine, du menthol, un liquide ou gel chauffant, des agents sensibilisants, des agents désensibilisants, ou des spermicides.

3. Le préservatif revêtu de la revendication 1, sachant que, lors de l'application du revêtement fluide, de l'enroulement, et de l'emballage séquentiels du préservatif, le matériau de revêtement fluide recouvre complètement sensiblement la totalité de la surface extérieure de la gaine tubulaire immédiatement ou dans un délai de quelques heures.

4. Le préservatif revêtu de la revendication 1, sachant que 50 à 1000 mg du matériau de revêtement fluide sont disposés sur la gaine tubulaire.

5. Le préservatif revêtu de la revendication 1, sachant que la composition polymérique comprend au moins l'un d'un latex de caoutchouc naturel, de polyisoprènes synthétiques, de polychloroprènes, de nitriles de butadiène carboxylés, de polyuréthanes ou de copolymères de polyuréthane-polyurée, ou de mélanges de ceux-ci.

6. Procédé de formation du préservatif (100) revêtu emballé de l'une quelconque des revendications 1 à 5, comprenant :
le trempage d'une forme de préservatif dans une émulsion polymérique pour former une couche de l'émulsion polymérique sur la forme de préservatif ;
le durcissement de la couche d'émulsion polymérique pour former un préservatif (100) non enroulé ;
l'application d'un matériau de revêtement fluide sur le préservatif (100) non enroulé à un ou plusieurs emplacements de revêtement le long d'une gaine tubulaire (116) du préservatif (100) de telle sorte qu'une surface extérieure (114) du préservatif (100) non enroulé ne soit pas complètement recouverte, sachant que le matériau de revêtement fluide comprend un ou plusieurs lubrifiants à base de silicone ;
l'enroulement du préservatif (100) non enroulé en une configuration roulée, sachant que le matériau de revêtement fluide migre de telle sorte que le matériau de revêtement fluide recouvre complètement sensiblement la totalité d'une surface extérieure du préservatif (100) revêtu ; et
l'emballage du préservatif (100) complètement revêtu dans un emballage en feuille.

7. Le procédé de la revendication 6, sachant que le préservatif revêtu inclut une gaine tubulaire ayant une pointe à une extrémité fermée de la gaine tubulaire et une extrémité ouverte opposée à l'extrémité fermée sur la gaine tubulaire.

8. Le procédé de la revendication 7, sachant que l'enroulement du préservatif pousse le matériau de revêtement fluide, forçant le matériau de revêtement fluide à migrer de l'extrémité ouverte de la gaine tubulaire vers la pointe à l'extrémité fermée de la gaine tubulaire.

9. Le procédé de la revendication 6, sachant que le matériau de revêtement fluide est appliqué sur un emplacement de revêtement unique le long d'une gaine tubulaire du préservatif non enroulé.

10. Le procédé de la revendication 6, sachant que le matériau de revêtement fluide est appliqué sur le préservatif non enroulé par l'un d'une pulvérisation, d'un trempage, d'une application à l'éponge, d'un roulement dans le matériau de revêtement fluide, ou d'une application dosée.

11. Le procédé de la revendication 6, sachant que l'émulsion comprend au moins l'un d'un latex de caoutchouc naturel, de polyisoprènes synthétiques, de polychloroprènes, de nitriles de butadiène carboxylés, de polyuréthanes ou de copolymères de polyuréthane-polyurée, ou de mélanges de ceux-ci.

12. Le procédé de la revendication 6, comprenant en outre la disposition de 50 à 1000 mg du matériau de revêtement fluide sur le préservatif.

13. Le procédé de la revendication 6, sachant que lors de l'application du revêtement fluide, de l'enroulement, et de l'emballage séquentiels du préservatif, le matériau de revêtement fluide recouvre complètement sensiblement la totalité de la surface extérieure de la gaine tubulaire immédiatement ou dans un délai de quelques heures.
